# FASCICULE DE BREVET EUROPEEN

(11) **EP 2 012 701 B1**
(45) Date de publication et mention de la délivrance du brevet: **08.06.2011**
(21) Numéro de dépôt: 07731263.5
(22) Date de dépôt: 06.04.2007
(51) Int. Cl.: A61B 19/02, B65D 75/20, B65D 65/14, B65D 75/58

(54) **EMBALLAGE PELABLE POUR LA STÉRILISATION**
ABZIEHPACKUNG ZUR STERILISATION
PEEL-OFF PACKAGE FOR STERILIZATION

(30) Priorité: 06.04.2006 FR 0603054
(43) Date de publication de la demande: 14.01.2009
(73) Titulaire: Amcor Flexibles SPS, 77120 Coulommiers (FR)
(72) Inventeur: SOUARD, Sylvère, 77600 Conches-sur-Gondoire (FR)
(74) Mandataire: Niemann, Frédéric
(86) Numéro de dépôt international: PCT/FR2007/000590
(87) Numéro de publication internationale: WO 2007/116139

(56) Documents cités:
- DE-U1- 20 113 173
- GB-A- 988 693
- US-A- 3 326 450

## Description

L'invention porte sur un emballage pelable pour la stérilisation.

L'état stérile est un état éphémère. La stérilité d'un objet ne peut se concevoir que dans le cadre de la protection de cet état. Le conditionnement est une étape capitale dans le processus de l'obtention de l'état stérile. Les moyens de conditionnement sont conçus pour résister aux contraintes habituelles de stérilisation, de transport, et de stockage pour permettre une manipulation et une ouverture facile.

Plusieurs techniques existent pour stériliser les dispositifs médicaux et en particulier les instruments destinés à être en contact avec le corps humain, notamment les instruments chirurgicaux. Parmi ces techniques on peut citer les techniques les plus couramment utilisées que sont la stérilisation par l'oxyde d'éthylène, la stérilisation vapeur et la stérilisation par gaz plasma.

Au cours du procédé de stérilisation, la pièce à stériliser ou le plateau d'instruments contenant les pièces à stériliser sont d'abord enveloppés dans un matériau dont une des fonctions est de maintenir la stérilité du produit une fois l'étape de stérilisation terminée. Ce matériau doit empêcher toute contamination ultérieure sans réduire l'efficacité de l'étape de stérilisation. Le choix du matériau est ainsi dicté par les contraintes propres à chaque technique de stérilisation.

Deux techniques sont couramment utilisées en milieu médical pour envelopper les pièces à stériliser ou les plateaux d'instruments contenant les pièces à stériliser.

La première consiste à envelopper l'objet à stériliser avec un matériau adapté au procédé de stérilisation en adoptant la technique de pliage « Pasteur ». Selon cette technique, aucun scellage n'est réalisé pour fermer l'emballage, un pliage répété du matériau permet d'obtenir une tortuosité suffisante pour maintenir la stérilité du contenu de l'emballage. Cette technique est particulièrement adaptée pour la stérilisation des objets volumineux et/ou lourds. Cependant si le pliage est de mauvaise qualité, la stérilité de l'objet ne sera pas maintenue dans le temps. De la qualité du pliage dépend le maintien de la stérilité. Cette technique présente donc le désavantage de dépendre de manière très importante de l'intervention humaine.

La deuxième technique consiste à mettre l'objet à stériliser dans un emballage pelable. Une fois l'objet dans l'emballage, l'emballage est scellé, puis soumis à l'étape de stérilisation. Les emballages sont le plus souvent constitués de deux feuilles distinctes constituées par des matériaux différents. Une des feuilles est le plus souvent transparente. Les emballages sont adaptés à la technique de stérilisation utilisée. Les emballages pelables étant constitués de deux feuilles thermoscellées, la résistance mécanique peut être limitée par la résistance mécanique de la soudure qui assure la jonction entre les deux feuilles, ou par la résistance de l'emballage lui-même.

Un des buts d'un mode de réalisation de la présente invention est de fournir un emballage pelable pour la stérilisation qui soit adapté aux objets volumineux et/ou lourds afin de diminuer le plus possible l'intervention humaine, et ainsi les risques de contamination ultérieure.

Un mode de réalisation de la présente invention est un support adapté à au moins un mode de stérilisation et au maintien de la stérilité comprenant sur une de ses faces un adhésif thermoscellable, ledit adhésif formant une bande qui décrit une courbe, ladite courbe étant symétrique par rapport à un axe, ledit axe étant l'axe de pliage du support, ledit support une fois plié et thermoscellé pouvant constituer un emballage pelable pour la stérilisation.

Selon un autre mode de réalisation de l'invention, l'invention concerne un support adapté à au moins un mode de stérilisation et au maintien de la stérilité, comprenant sur une de ses faces un adhésif thermoscellable, ledit adhésif formant deux bandes qui décrivent deux courbes, lesdites courbes étant symétriques l'une par rapport à l'autre par rapport à un axe, ledit axe étant l'axe de pliage du support, ledit support une fois plié, découpé et thermoscellé pouvant constituer un emballage pelable pour la stérilisation.

D'après l'invention, ces deux modes de réalisations sont caractérisés en ce que le support est constitué d'un matériau constitué par un polymère ou un mélange de polymères choisis dans le groupe constitué par le polyéthylène de haute densité, le polypropylène, le polyamide, le polyester, et en ce que l'adhésif thermoscellable est un adhésif qui présente une liaison adhésif-adhésif plus faible que la liaison adhésif-support, l'adhésif thermoscellable étant un adhésif à base aqueuse et étant déposé sur une zone du support enduit avec un agent hydrophobe.

Par emballage, on entend tout ce qui sert à emballer, il peut s'agir par exemple d'un sac, d'un sachet, d'une poche, d'une pochette.

Par emballage pelable, on entend un emballage qui est susceptible de s'ouvrir au niveau de la jonction thermoscellée suite à une force exercée par l'utilisateur. L'ouverture se fait par rupture de la liaison adhésif-adhésif.

Typiquement ladite ou lesdites courbe(s) est (sont) fermée(s) ou non fermé(s). Lorsque ladite ou lesdites courbe (s) est (sont) non fermée(s), l'étanchéité de l'emballage sera assurée en thermoscellant directement le support sur lui-même. Pour ce faire, l'homme du métier choisira des supports constitués de matériaux qui permettent au support d'être thermoscellé sur lui-même. De tels matériaux sont par exemple le SMS et le SMMS.

Typiquement l'aire de la surface définie par la ou les courbes pourra être variable et sera fonction de la taille des pièces que l'on souhaite stériliser. Typiquement l'aire pourra être comprise entre 100 cm² et 20 000 cm², en particulier entre 1000 et 10 000 cm². Le support est constitué d'un matériau qui permet de maintenir la stérilité du produit une fois l'étape de stérilisation terminée. Ce matériau empêche toute contamination ultérieure sans réduire l'efficacité de l'étape de stérilisation. Le matériau présente une perméabilité suffisante à tous les agents physiques et/ou chimiques qui ont une influence sur l'efficacité du procédé de stérilisation. De plus le matériau permet l'évacuation de ces agents après la stérilisation. Typiquement, le support est constitué d'un matériau adapté à au moins un mode de stérilisation choisi parmi la stérilisation vapeur, la stérilisation par l'oxyde d'éthylène et la stérilisation par gaz plasma.

Selon un mode de réalisation particulier, le support est constitué d'un matériau adapté à au moins deux modes de stérilisation choisis parmi la stérilisation vapeur, la stérilisation par l'oxyde d'éthylène et la stérilisation par gaz plasma. L'utilisation de tels matériaux permet d'obtenir un emballage pelable « universel », c'est-à-dire adapté aux principales techniques de stérilisation.

Selon un mode de réalisation préféré, le support est constitué d'un matériau adapté à la stérilisation vapeur.

Préférentiellement le matériau est non tissé. Typiquement le matériau présente une structure de type SMS (spunbond-meltblown-spunbond) ou SMMS (spunbond-meltblown-meltblown-spunbond). Alternativement le matériau est de type Tyvek^{®} ou Dextex^{®}. Tous ces matériaux sont connus pour être adaptés à la stérilisation du matériel médical et chirurgical.

L'adhésif thermoscellable utilisé pour la présente invention présente avantageusement une liaison adhésif-adhésif plus faible que la liaison adhésif-support de façon à minimiser l'émission de fibres lors de l'ouverture de l'emballage.

A titre d'exemple d'adhésifs à base aqueuse, que l'homme du métier pourra utiliser, on peut citer : les résines hydrocarbonées et styréniques, les émulsions vinyliques, les dispersions de copolymères d'ethylène, les cires.

Typiquement l'adhésif thermoscellable pourra être déposé sur le support à température ambiante.

Selon l'invention afin de réduire d'éventuel problème de perméabilité induit par l'utilisation d'adhésif à base aqueuse, l'adhésif est déposé sur une zone du support enduite avec un agent hydrophobe. A titre d'exemple d'agent hydrophobe, on peut citer les résines thermoplastiques hydrocarbonées. La zone enduite pourra couvrir toute une face du support ou seulement couvrir une bande du support.

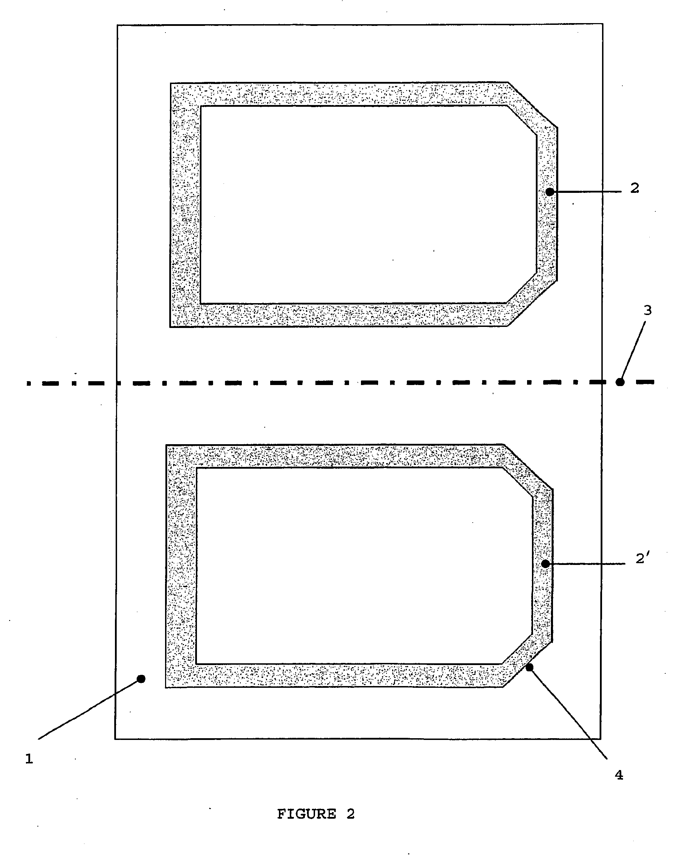

Selon un mode de réalisation particulier, la courbe fermée présente une forme essentiellement rectangulaire. Typiquement, afin de faciliter l'ouverture de l'emballage par l'utilisateur, la forme rectangulaire pourra être modifiée de façon à prévoir une ou plusieurs pattes de pelabilité. Par exemple, un ou deux angles de la forme rectangulaire pourront être tronqués (cf. figure 2). Avantageusement, la forme rectangulaire pourra présenter des coins arrondis afin de répartir au mieux toutes les éventuelles contraintes mécaniques induites par le contenu de l'emballage sur la jonction scellée.

L'adhésif thermoscellable peut être déposé en bande suivant la ou les courbes telles que décrites ci-dessus sur le support selon toutes les techniques usuelles, par exemple le dépôt en zone selon un procédé par héliographie, par planographie ou par flexographie. Typiquement la largeur de la bande d'adhésif déposée sur le support peut varier le long de la courbe. Typiquement, il peut être prévu que pour la partie de la bande d'adhésif qui sera thermoscellée par l'utilisateur final, la largeur de la bande sera 1,2 à 3 fois plus large que la largeur maximale de la bande sur le reste de la courbe. Ainsi le scellement par l'utilisateur final de l'emballage est facilité.

En fonction de la résistance mécanique souhaitée après stérilisation pour la soudure qui assure la fermeture de l'emballage, l'homme du métier choisira un adhésif thermoscellable adapté au mode de stérilisation et fera varier la largeur de la bande d'adhésif et la quantité d'adhésif déposée sur le support. Typiquement la résistance mécanique de la soudure sera comprise entre 400g et 600g.

Pour déterminer la résistance mécanique de la soudure, on pourra utiliser la méthode de contrôle dynamomètre. Typiquement la largeur de la bande pourra être comprise entre 0,5 et 3 cm et la quantité déposée pourra être comprise entre 10 et 30 g/m².

La géométrie du dépôt de l'adhésif thermoscellable permet, lorsque le support est plié, de réaliser des soudures de très bonnes qualités.

Un mode de réalisation de l'invention concerne un emballage pelable pour la stérilisation comprenant un support tel que décrit précédemment comprenant sur une de ses faces un adhésif thermoscellable, ledit adhésif formant une bande qui décrit une courbe, ladite courbe étant symétrique par rapport à un axe, ledit axe étant l'axe de pliage du support, caractérisé en ce que le support est plié et thermoscellé partiellement de sorte que l'emballage pelable présente une ouverture thermoscellable destinée à permettre le positionnement à l'intérieur de l'emballage des pièces à stériliser. Cet emballage pelable pour la stérilisation est particulièrement adapté aux objets volumineux ét/ou lourds, car la résistance mécanique de l'emballage est accrue car l'emballage est constitué d'un seul tenant. De plus comme l'emballage présente un pli, la charge des objets lourds est supportée par le pli et non pas uniquement par la jonction thermoscellée.

Un autre mode de réalisation de l'invention concerne un emballage pelable pour la stérilisation comprenant un support tel que décrit précédemment comprenant sur une de ses faces un adhésif thermoscellable, ledit adhésif formant deux bandes qui décrivent deux courbes, lesdites courbes étant symétriques l'une par rapport à l'autre par rapport à un axe, ledit axe étant l'axe de pliage du support, caractérisé en ce que le support est plié, découpé et thermoscellé partiellement de sorte que l'emballage pelable présente une ouverture thermoscellable destinée à permettre le positionnement à l'intérieur de l'emballage des pièces à stériliser. L'absence de pli facilite l'ouverture de l'emballage par l'utilisateur.

Un mode de réalisation de l'invention concerne un ensemble d'emballages pelables comprenant un premier emballage pelable tel que décrit précédemment compris dans un deuxième emballage tel que décrit précédemment, les deux emballages étant adaptés au même mode de stérilisation.

Typiquement le deuxième emballage peut servir d'emballage de transport qui pourra être retiré juste avant l'entrée dans une pièce stérile telle qu'une salle d'opération. Typiquement le deuxième emballage pourra être retiré dans l'éventuel sas qui précède la salle stérile. Ainsi la stérilité de la surface extérieure du premier emballage est préservée le plus longtemps possible.

Selon un mode de réalisation particulier les deux emballages sont adaptés à la stérilisation vapeur, le premier emballage est constitué d'un matériau non tissé qui présente une structure de type SMS ou SMMS, le deuxième emballage est constitué de cellulose. La résistance mécanique de l'ensemble d'emballages est assurée principalement par le premier emballage constitué d'un matériau non tissé. L'utilisation d'un deuxième emballage en cellulose facilite l'évacuation de la vapeur d'eau.

Un mode de réalisation de la présente invention est un procédé de fabrication d'un emballage pelable tel que décrit précédemment comprenant les étapes suivantes :
a) fournir un support adapté à au moins un mode de stérilisation et au maintien de la stérilité ;
b) déposer une bande d'adhésif thermoscellable sur une des faces du support de façon à ce que la bande décrive une courbe, ladite courbe étant symétrique par rapport à un axe, ledit axe étant l'axe de pliage du support ;
c) plier le support selon ledit axe ;
   - thermosceller partiellement le support plié de sorte que l'emballage pelable obtenu présente une ouverture thermoscellable destinée à permettre le positionnement à l'intérieur de l'emballage de pièces à stériliser.

Un autre mode de réalisation est un procédé de fabrication d'un emballage pelable pour la stérilisation tel que décrit précédemment comprenant les étapes suivantes :
a) fournir un support adapté à au moins un mode de stérilisation et au maintien de la stérilité ;
b) déposer deux bandes d'adhésif thermoscellable sur une des faces du support de façon à ce que les bandes décrivent deux courbes, lesdites courbes étant symétriques l'une par rapport à l'autre par rapport à un axe, ledit axe étant l'axe de pliage du support ;
c) plier le support selon ledit axe ;
d) découper le support de façon à retirer le pli ;
e) thermosceller partiellement le support plié de sorte que l'emballage pelable obtenu présente une ouverture thermoscellable destinée à permettre le positionnement à l'intérieur de l'emballage des pièces à stériliser, l'étape e) pouvant être réalisée avant ou simultanément avec l'étape d).

Un mode de réalisation de la présente invention est un procédé de stérilisation d'une pièce comprenant les étapes suivantes :
a) introduire la pièce à stériliser dans un emballage pelable pour la stérilisation tel que décrit précédemment ou dans un ensemble d'emballages pelables tel que décrit précédemment;
b) thermosceller le ou les emballages ;
c) stériliser le contenu de l'emballage ou de l'ensemble d'emballages.

Typiquement les pièces à stériliser sont des dispositifs médicaux disposés sur un plateau à instruments.

Un mode de réalisation particulier de l'invention concerne un procédé de stérilisation tel que décrit précédemment caractérisé en ce que la stérilisation est une stérilisation vapeur.

Un mode de réalisation de l'invention concerne l'utilisation en milieu hospitalier d'un emballage tel que décrit précédemment ou d'un ensemble d'emballages pelables tel que décrit précédemment.

Un mode de réalisation de l'invention concerne un procédé de fabrication d'un support tel que décrit précédemment comprenant les étapes suivantes :
a) fournir un support adapté à au moins un mode de stérilisation et au maintien de la stérilité ;
b) déposer une bande d'adhésif thermoscellable sur une des faces du support de façon à ce que la bande décrive une courbe, ladite courbe étant symétrique par rapport à un axe, ou bien
b') déposer deux bandes d'adhésif thermoscellable sur une des faces du support de façon à ce que les bandes décrivent deux courbes, lesdites courbes étant symétriques l'une par rapport à l'autre par rapport à un axe.

Le support peut avoir n'importe quelle forme ou dimension, le procédé de fabrication s'adapte à toutes les dimensions d'emballages souhaitées.

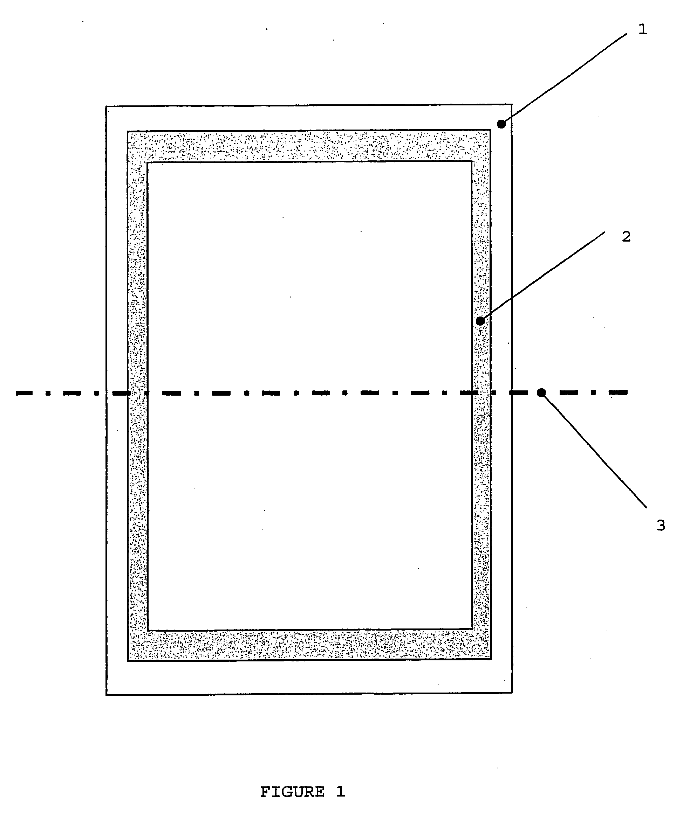

La figure 1 représente de manière schématique un mode de réalisation de l'invention. Il s'agit d'une vue de dessus du support (1) sur lequel a été déposée une bande d'adhésif thermoscellable (2). L'axe de pliage et de symétrie (3) est représenté en pointillé.
La figure 2 représente de manière schématique un autre mode de réalisation de l'invention. Il s'agit d'une vue de dessus du support (1) sur lequel ont été déposées deux bandes d'adhésif thermoscellable (2 et 2'). L'axe de pliage et de symétrie (3) est représenté en pointillé. Deux pattes de pelabilité (4) sont représentées.

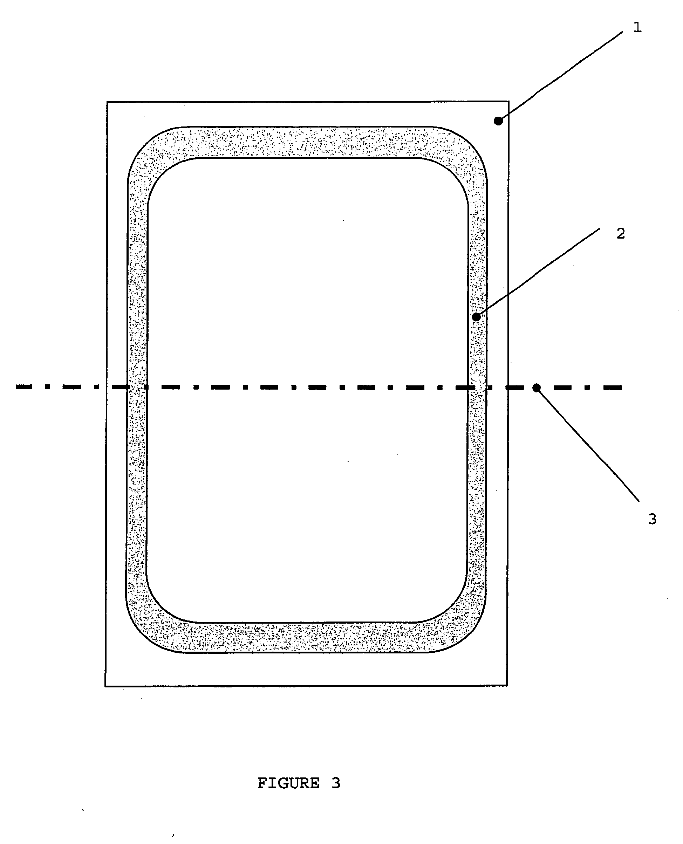

La figure 3 représente de manière schématique un mode de réalisation dans le quel les pattes de pelabilité (4) présentent une forme arrondie.

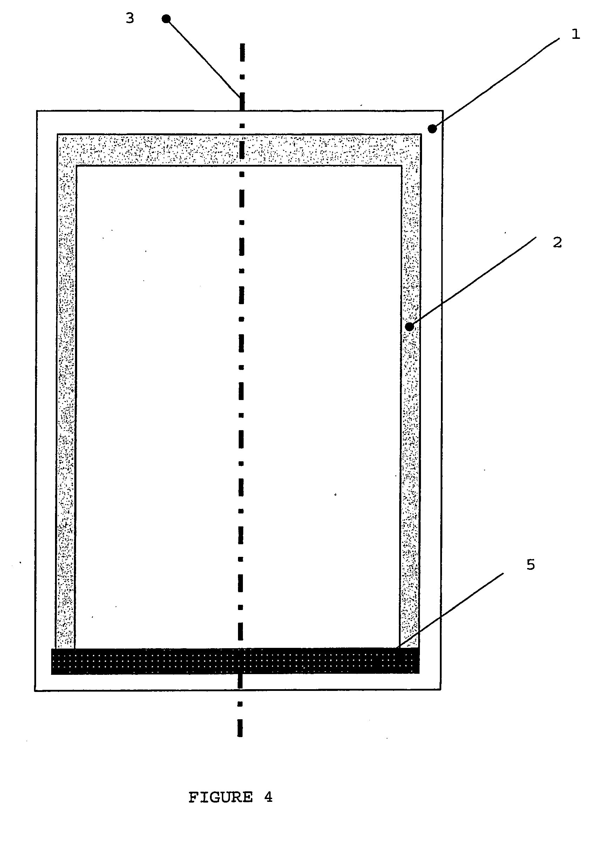

La figure.4 représente de manière schématique un mode de réalisation de l'invention. La zone (5) du support correspond à la zone du support qui sera thermoscellée sur elle-même.

## Revendications

1. Support (1) adapté à au moins un mode de stérilisation et au maintien de la stérilité comprenant sur une de ses faces un adhésif thermoscellable, ledit adhésif formant une bande (2) qui décrit une courbe, ladite courbe étant symétrique par rapport à un axe (3), ledit axe étant l'axe de pliage du support, ledit support une fois plié et thermoscellé pouvant constituer un emballage pelable pour la stérilisation, **caractérisé en ce que** le support est constitué d'un matériau constitué par un polymère ou un mélange de polymères choisis dans le groupe constitué par le polyéthylène de haute densité, le polypropylène, le polyamide, le polyester,
et **en ce que** l'adhésif thermoscellable est un adhésif qui présente une liaison adhésif-adhésif plus faible que la liaison adhésif-support, l'adhésif thermoscellable étant un adhésif à base aqueuse et étant déposé sur une zone du support enduite avec un agent hydrophobe.

2. Support (1) adapté à au moins un mode de stérilisation et au maintien de la stérilité, comprenant sur une de ses faces un adhésif thermoscellable (2), ledit adhésif formant deux bandes (2 et 2') qui décrivent deux courbes, lesdites courbes étant symétriques l'une par rapport à l'autre par rapport à un axe (3), ledit axe étant l'axe de pliage du support, ledit support une fois plié, découpé et thermoscellé pouvant constituer un emballage pelable pour la stérilisation, **caractérisé en ce que** le support est constitué d'un matériau constitué par un polymère ou un mélange de polymères choisis dans le groupe constitué par le polyéthylène de haute densité, le polypropylène, le polyamide, le polyester,
et **en ce que** l'adhésif thermoscellable est un adhésif qui présente une liaison adhésif-adhésif plus faible que la liaison adhésif-support, l'adhésif thermoscellable étant un adhésif à base aqueuse et étant déposé sur une zone du support enduite avec un agent hydrophobe.

3. Support selon la revendication 1 ou 2, **caractérisé en ce que** ladite ou lesdites courbe(s) est (sont) fermée(s).

4. Support selon l'une quelconque des revendications 1 à 3, **caractérisé en ce que** le support est constitué d'un matériau adapté à au moins deux modes de stérilisation choisi parmi la stérilisation vapeur, la stérilisation par l'oxyde d'éthylène et la stérilisation par gaz plasma.

5. Support selon l'une quelconque des revendications précédentes, **caractérisé en ce que** ledit matériau est non tissé et présente une structure de type SMS ou SMMS.

6. Emballage pelable pour la stérilisation comprenant un support selon l'une quelconque des revendications 1, 3 à 5, comprenant sur une de ses faces un adhésif thermoscellable, ledit adhésif formant une bande (2) qui décrit une courbe, ladite courbe étant symétrique par rapport à un axe (3), ledit axe étant l'axe de pliage du support, **caractérisé en ce que** le support est plié et thermoscellé partiellement de sorte que l'emballage pelable présente une ouverture thermoscellable destinée à permettre le positionnement à l'intérieur de l'emballage des pièces à stériliser.

7. Emballage pelable pour la stérilisation comprenant un support selon l'une quelconque des revendications 2 à 5, comprenant sur une de ses faces un adhésif thermoscellable (2), ledit adhésif formant deux bandes (2 et 2') qui décrivent deux courbes, lesdites courbes étant symétriques l'une par rapport à l'autre par rapport à un axe (3), ledit axe étant l'axe de pliage du support, **caractérisé en ce que** le support est plié, découpé et thermoscellé partiellement de sorte que l'emballage pelable présente une ouverture thermoscellable destinée à permettre le positionnement à l'intérieur de l'emballage des pièces à stériliser.

8. Ensemble d'emballages pelables comprenant un premier emballage pelable selon la revendication 6 ou 7 compris dans un deuxième emballage selon la revendication 6 ou 7, les deux emballages étant adaptés au même mode de stérilisation.

9. Ensemble d'emballages pelables comprenant un premier emballage pelable selon la revendication 6 ou 7 compris dans un deuxième emballage, dans lequel les deux emballages sont adaptés à la stérilisation vapeur, le premier emballage est constitué d'un matériau non tissé qui présente une structure de type SMS ou SMMS, le deuxième emballage est constitué de cellulose.

10. Procédé de fabrication d'un emballage pelable pour la stérilisation selon la revendication 6 comprenant les étapes suivantes :
a) fournir un support adapté à au moins un mode de stérilisation et au maintien de la stérilité ;
b) déposer une bande d'adhésif thermoscellable sur une des faces du support de façon à ce que la bande décrive une courbe, ladite courbe étant symétrique par rapport à un axe, ledit axe étant l'axe de pliage du support ;
c) plier le support selon ledit axe ;
- thermosceller partiellement le support plié de sorte que l'emballage pelable obtenu présente une ouverture thermoscellable destinée à permettre le positionnement à l'intérieur de l'emballage de pièces à stériliser.

11. Procédé de fabrication d'un emballage pelable pour la stérilisation selon la revendication 7 comprenant les étapes suivantes :
a) fournir un support adapté à au moins un mode de stérilisation et au maintien de la stérilité ;
b) déposer deux bandes d'adhésif thermoscellable sur une des faces du support de façon à ce que les bandes décrivent deux courbes, lesdites courbes étant symétriques l'une par rapport à l'autre par rapport à un axe, ledit axe étant l'axe de pliage du support ;
c) plier le support selon ledit axe ;
d) découper le support de façon à retirer le pli ;
e) thermosceller partiellement le support plié de sorte que l'emballage pelable obtenu présente une ouverture thermoscellable destinée à permettre le positionnement à l'intérieur de l'emballage des pièces à stériliser, l'étape e) pouvant être réalisée avant ou simultanément avec l'étape d).

12. Procédé de stérilisation d'une pièce comprenant les étapes suivantes :
a) introduire la pièce à stériliser dans un emballage pelable pour la stérilisation selon la revendication 6 ou 7 ou dans un ensemble d'emballages pelables selon la revendication 8 ou 9 ;
b) thermosceller le ou les emballages ;
c) stériliser le contenu de l'emballage ou de l'ensemble d'emballages.

13. Utilisation en milieu hospitalier d'un emballage selon la revendication 6 ou 7 ou d'un ensemble d'emballages pelables selon la revendication 8 ou 9.

14. Procédé de fabrication d'un support selon l'une quelconque des revendications 1 à 5 comprenant les étapes suivantes :
a) fournir un support adapté à au moins un mode de stérilisation et au maintien de la stérilité ;
b) déposer une bande d'adhésif thermoscellable sur une des faces du support de façon à ce que la bande décrive une courbe, ladite courbe étant symétrique par rapport à un axe, ou bien
b') déposer deux bandes d'adhésif thermoscellable sur une des faces du support de façon à ce que les bandes décrivent deux courbes, lesdites courbes étant symétriques l'une par rapport à l'autre par rapport à un axe.

## Claims

1. Support (1) suited to at least one sterilization method and to sterility maintenance, comprising on one of its faces a heat-sealable adhesive, said adhesive forming a strip (2) which describes a curve, said curve being symmetrical with respect to an axis (3), said axis being the axis of folding of the support, said support once folded and heat-sealed being capable of constituting a peelable sterilization pack,
**characterized in that** the support is constituted by a material constituted by a polymer or a mixture of polymers chosen from the group constituted by high density polyethylene, polypropylene, polyamide and polyester,
and **in that** the heat-sealable adhesive is an adhesive which has an adhesive-adhesive bond which is weaker than the adhesive-support bond, the heat-sealable adhesive being a water-based adhesive and being applied to a area of the support coated with a hydrophobic agent.

2. Support (1) suited to at least one sterilization method and to sterility maintenance, comprising on one of its faces a heat-sealable adhesive (2), said adhesive forming two strips (2 and 2') which describe two curves, said curves being symmetrical in relation to each other with respect to an axis (3), said axis being the axis of folding of the support, said support once folded, trimmed and heat-sealed being able to constitute a peelable sterilization pack, **characterized in that** the support is constituted by a material constituted by a polymer or a mixture of polymers chosen from the group constituted by high density polyethylene, polypropylene, polyamide and polyester,
and **in that** the heat-sealable adhesive is an adhesive which has an adhesive-adhesive bond which is weaker than the adhesive-support bond, the heat-sealable adhesive being a water-based adhesive and being applied to a area of the support coated with a hydrophobic agent.

3. Support according to claim 1 or 2, **characterized in that** said curve(s) is (are) closed.

4. Support according to any one of claims 1 to 3, **characterized in that** the support is constituted by a material suited to at least two sterilization methods chosen from steam sterilization, ethylene oxide sterilization and plasma gas sterilization.

5. Support according to any one of the preceding claims, **characterized in that** said material is nonwoven and has a structure of SMS or SMMS type.

6. Peelable sterilization pack comprising a support according to any one of claims 1, 3 to 5, comprising on one of its faces a heat-sealable adhesive, said adhesive forming a strip (2) which describes a curve, said curve being symmetrical with respect to an axis (3), said axis being the axis of folding of the support, **characterized in that** the support is folded and partially heat-sealed such that the peelable pack has a heat-sealable opening intended to allow the items for sterilization to be positioned inside the packaging.

7. Peelable sterilization pack comprising a support according to any one of claims 2 to 5, comprising on one of its faces a heat-sealable adhesive (2), said adhesive forming two strips (2 and 2') which describe two curves, said curves being symmetrical to each other with respect to an axis (3), said axis being the axis of folding of the support, **characterized in that** the support is folded, trimmed and partially heat-sealed such that the peelable pack has a heat-sealable opening intended to allow the items for sterilization to be positioned inside the packaging.

8. Set of peelable packs comprising a first peelable pack according to claim 6 or 7 contained in a second pack according to claim 6 or 7, the two packs being suited to the same sterilization method.

9. Set of peelable packs comprising a first peelable pack according to claim 6 or 7 contained in a second pack, in which the two packs are suited to steam sterilization, the first pack is constituted by a nonwoven material which has a structure of SMS or SMMS type, the second pack is constituted by cellulose.

10. Process for producing a peelable sterilization pack according to claim 6 comprising the following steps:
a) supplying a support suited to at least one sterilization method and to sterility maintenance;
b) applying a strip of heat-sealable adhesive to one of the faces of the support such that the strip describes a curve, said curve being symmetrical with respect to an axis, said axis being the axis of folding of the support;
c) folding the support along said axis;
- partially heat-sealing the folded support such that the peelable pack obtained has a heat-sealable opening intended to allow the items for sterilization to be positioned inside the pack.

11. Process for the production of a peelable sterilization pack according to claim 7 comprising the following steps:
a) supplying a support suited to at least one sterilization method and to sterility maintenance;
b) applying two strips of heat-sealable adhesive to one of the faces of the support such that the strips describe two curves, said curves being symmetrical to each other with respect to an axis, said axis being the axis of folding of the support;
c) folding the support along said axis;
d) trimming the support so as to remove the fold;
e) partially heat-sealing the folded support such that the peelable pack obtained has a heat-sealable opening intended to allow the items for sterilization to be positioned inside the packaging, wherein step e) may be carried out before or simultaneously with step d).

12. Method for the sterilization of an item comprising the following steps:
a) introducing the item to be sterilized into a peelable sterilization pack according to claim 6 or 7 or into a set of peelable packs according to claim 8 or 9;
b) heat-sealing the pack or packs;
c) sterilizing the contents of the pack or set of packs.

13. Use in a hospital environment of a pack according to claim 6 or 7 or a set of peelable packs according to claim 8 or 9.

14. Method for the production of a support according to any one of claims 1 to 5, comprising the following steps:
a) supplying a support suited to at least one sterilization method and to sterility maintenance;
b) applying a strip of heat-sealable adhesive to one of the faces of the support such that the strip describes a curve, said curve being symmetrical with respect to an axis, or
b') applying two strips of heat-sealable adhesive to one of the faces of the support such that the strips describe two curves, said curves being symmetrical to each other with respect to an axis.

## Patentansprüche

1. Basismaterial (1), das für mindestens eine Art der Sterilisierung und zur Aufrechterhaltung der Sterilität ausgelegt ist, aufweisend einen heißsiegelbaren Klebstoff auf einer seiner Flächen, wobei der Klebstoff einen Streifen (2) bildet, der eine Kurve beschreibt, wobei die Kurve bezüglich einer Achse (3) symmetrisch ist, wobei die Achse die Faltachse des Basismaterials ist, wobei das einmal gefaltete und heißgesiegelte Basismaterial eine abziehbare Verpackung für die Sterilisierung bilden kann,
**dadurch gekennzeichnet, dass** das Basismaterial aus einem Material besteht, das aus einem Polymer oder einem Gemisch von Polymeren besteht, die ausgewählt sind aus der Gruppe, die aus Polyethylen hoher Dichte, Polypropylen, Polyamid, Polyester besteht,
und **dadurch**, dass der heißsiegelbare Klebstoff ein Klebstoff ist, dessen Klebstoff-Klebstoff-Verbindung schwächer ist als die Klebstoff-Basismaterial-Verbindung, wobei der heißsiegelbare Klebstoff ein Klebstoff auf wässriger Basis ist und auf einen Bereich des Basismaterials aufgebracht ist, der mit einem hydrophoben Mittel beschichtet ist.

2. Basismaterial (1) das für mindestens eine Art der Sterilisierung und zur Aufrechterhaltung der Sterilität ausgelegt ist, aufweisend einen heißsiegelbaren Klebstoff (2) auf einer seiner Flächen, wobei der Klebstoff zwei Streifen (2 und 2') bildet, die zwei Kurven beschreiben, wobei die Kurven bezüglich einer Achse (3) zueinander symmetrisch sind, wobei die Achse die Faltachse des Basismaterials ist, wobei das einmal gefaltete, geschnittene und heißgesiegelte Basismaterial eine abziehbare Verpackung für die Sterilisierung bilden kann,
**dadurch gekennzeichnet, dass** das Basismaterial aus einem Material besteht, das aus einem Polymer oder einem Gemisch von Polymeren besteht, die ausgewählt sind aus der Gruppe, die aus Polyethylen hoher Dichte, Polypropylen, Polyamid, Polyester besteht,
und **dadurch**, dass der heißsiegelbare Klebstoff ein Klebstoff ist, dessen Klebstoff-Klebstoff-Verbindung schwächer ist als die Klebstoff-Basismaterial-Verbindung, wobei der heißsiegelbare Klebstoff ein Klebstoff auf wässriger Basis ist und auf einen Bereich des Basismaterials aufgebracht ist, der mit einem hydrophoben Mittel beschichtet ist.

3. Basismaterial nach Anspruch 1 oder 2, **dadurch gekennzeichnet, dass** die Kurve(n) in sich geschlossen ist (sind).

4. Basismaterial nach einem der Ansprüche 1 bis 3, **dadurch gekennzeichnet, dass** das Basismaterial aus einem Material besteht, das für mindestens zwei Arten der Sterilisierung ausgelegt ist, die ausgewählt sind aus der Dampfsterilisierung, der Sterilisierung mit Ethylenoxid und der Sterilisierung mit Plasmagas.

5. Basismaterial nach einem der vorangehenden Ansprüche, **dadurch gekennzeichnet, dass** das Material nicht gewebt ist und eine Struktur vom SMS-Tpy oder SMMS-Typ aufweist.

6. Abziehbare Verpackung zur Sterilisierung, aufweisend ein Basismaterial nach einem der Ansprüche 1, 3 bis 5, das einen heißsiegelbaren Klebstoff auf einer seiner Flächen aufweist, wobei der Klebstoff einen Streifen (2) bildet, der eine Kurve beschreibt, wobei die Kurve bezüglich einer Achse (3) symmetrisch ist, wobei die Achse die Faltachse des Basismaterials ist, **dadurch gekennzeichnet, dass** das Basismaterial dergestalt gefaltet und teilweise heißgesiegelt ist, dass die abziehbare Verpackung eine heißsiegelbare Öffnung aufweist, die dazu bestimmt ist, die Einbringung von zu sterilisierenden Teilen ins Innere der Verpackung zu erlauben.

7. Abziehbare Verpackung zur Sterilisierung, aufweisend ein Basismaterial nach einem der Ansprüche 2 bis 5, das einen heißsiegelbaren Klebstoff (2) auf einer seiner Flächen aufweist, wobei der Klebstoff zwei Streifen (2 und 2') bildet, die zwei Kurven beschreiben, wobei die Kurven bezüglich einer Achse (3) zueinander symmetrisch sind, wobei die Achse die Faltachse des Basismaterials ist, **dadurch gekennzeichnet, dass** das Basismaterial dergestalt gefaltet, geschnitten und teilweise heißgesiegelt ist, dass die abziehbare Verpackung eine heißsiegelbare Öffnung aufweist, die dazu bestimmt ist, die Einbringung von zu sterilisierenden Teilen ins Innere der Verpackung zu erlauben.

8. Set von abziehbaren Verpackungen, aufweisend eine erste abziehbare Verpackung nach Anspruch 6 oder 7, die enthalten ist in einer zweiten Verpackung nach Anspruch 6 oder 7, wobei die zwei Verpackungen für dieselbe Art der Sterilisierung ausgelegt sind.

9. Set von abziehbaren Verpackungen, aufweisend eine erste abziehbare Verpackung nach Anspruch 6 oder 7, die enthalten ist in einer zweiten Verpackung, bei dem die zwei Verpackungen für die Dampfsterilisierung ausgelegt sind, wobei die erste Verpackung aus einem nicht gewebten Material besteht, das eine Struktur vom SMS-Typ oder SMMS-Typ aufweist, wobei die zweite Verpackung aus Zellulose besteht.

10. Verfahren zur Herstellung einer abziehbaren Verpackung zur Sterilisierung nach Anspruch 6, folgende Schritte aufweisend:
a) Bereitstellen eines Basismaterials, das für mindestens eine Art der Sterilisierung und zur Aufrechterhaltung der Sterilität ausgelegt ist;
b) Aufbringen eines Streifens aus heißsiegelbarem Klebstoff auf eine der Flächen des Basismaterials dergestalt, dass der Streifen eine Kurve beschreibt, wobei die Kurve bezüglich einer Achse symmetrisch ist, wobei die Achse die Faltachse des Basismaterials ist;
c) Falten des Basismaterials entlang der Achse;
- teilweise Heißsiegeln des gefalteten Basismaterials dergestalt, dass die erhaltene abziehbare Verpackung eine heißsiegelbare Öffnung aufweist, die dazu bestimmt ist, die Einbringung von zu sterilisierenden Teilen ins Innere der Verpackung zu erlauben.

11. Verfahren zur Herstellung einer abziehbaren Verpackung zur Sterilisierung nach Anspruch 7, folgende Schritte aufweisend:
a) Bereitstellen eines Basismaterials, das für mindestens eine Art der Sterilisierung und zur Aufrechterhaltung der Sterilität ausgelegt ist;
b) Aufbringen von zwei Streifen aus heißsiegelbaren Klebstoff auf eine der Flächen des Basismaterials dergestalt, dass die Streifen zwei Kurven beschreiben, wobei die Kurven bezüglich einer Achse zueinander symmetrisch sind, wobei die Achse die Faltachse des Basismaterials ist;
c) Falten des Basismaterials entlang der Achse;
d) Zuschneiden des Basismaterials dergestalt, dass die Falte herausgenommen wird;
e) teilweise Heißsiegeln des gefalteten Basismaterials dergestalt, dass die erhaltene abziehbare Verpackung eine heißsiegelbare Öffnung aufweist, die dazu bestimmt ist, die Einbringung von zu sterilisierenden Teilen ins Innere der Verpackung zu erlauben, wobei Schritt e) vor oder gleichzeitig mit Schritt d) durchgeführt werden kann.

12. Verfahren zur Sterilisierung eines Teils, folgende Schritte aufweisend:
a) Einführen des zu sterilisierenden Teils in eine abziehbare Verpackung zur Sterilisierung nach Anspruch 6 oder 7, oder in ein Set von abziehbaren Verpackungen nach Anspruch 8 oder 9;
b) Heißsiegeln der Verpackung oder der Verpackungen;
c) Sterilisieren des Inhalts der Verpackung oder des Sets von Verpackungen.

13. Verwendung einer Verpackung nach Anspruch 6 oder 7 oder eines Sets von abziehbaren Verpackungen nach Anspruch 8 oder 9 im Umfeld eines Krankenhauses.

14. Verfahren zur Herstellung eines Basismaterials nach einem der Ansprüche 1 bis 5, folgende Schritte aufweisend:
a) Bereitstellen eines Basismaterials, das für mindestens eine Art der Sterilisierung und zur Aufrechterhaltung der Sterilität ausgelegt ist;
b) Aufbringen eines Streifens aus heißsiegelbarem Klebstoff auf eine der Flächen des Basismaterials dergestalt, dass der Streifen eine Kurve beschreibt, wobei die Kurve bezüglich einer Achse symmetrisch ist, oder
b') Aufbringen von zwei Streifen aus heißsiegelbarem Klebstoff auf eine der Flächen des Basismaterials dergestalt, dass die Streifen zwei Kurven beschreiben, wobei die Kurven bezüglich einer Achse zueinander symmetrisch sind.
